# EUROPÄISCHE PATENTSCHRIFT

(11) **EP 3 293 192 B1**
(45) Veröffentlichungstag und Bekanntmachung des Hinweises auf die Patenterteilung: **14.11.2018**
(21) Anmeldenummer: 16187584.4
(22) Anmeldetag: 07.09.2016
(51) Int. Cl.: C07F 9/6574, B01J 31/16, C07B 41/06, C07F 15/00, C07C 45/50

(54) **PHOSPHITE MIT EINEM DIHYDROXYTERPHENYL MIT EINEM TETRAPHENYL-DIOXAPHOSPHOLAN**
PHOSPHITES WITH A DIHYDROXYTERPHENYL WITH A TETRAPHENYL DIOXAPHOSPHOLANE
PHOSPHITE COMPRENANT UN TERPHÉNYLE DIHYDROXY COMPRENANT UN DIOXAPHOSPHOLANE-TÉTRAPHÉNYL

(43) Veröffentlichungstag der Anmeldung: 14.03.2018
(73) Patentinhaber: Evonik Degussa GmbH, 45128 Essen (DE)
(72) Erfinder: BÖRNER, Armin, 18059 Rostock (DE); DYBALLA, Katrin Marie, 45657 Recklinghausen (DE); FRANKE, Robert, 45772 Marl (DE); SELENT, Detlef, 18059 Rostock (DE)

(56) Entgegenhaltungen:
- EP-A1- 3 029 047
- EP-A1- 3 029 056
- DATABASE CA [Online] CHEMICAL ABSTRACTS SERVICE, COLUMBUS, OHIO, US; 2015, GANGOPADHYAY, ADITI ET AL: "Identification of inhibitors against the potential ligandable sites in the active cholera toxin", XP002767837, gefunden im STN Database accession no. 2015:247722 & GANGOPADHYAY, ADITI ET AL: "Identification of inhibitors against the potential ligandable sites in the active cholera toxin", COMPUTATIONAL BIOLOGY AND CHEMISTRY , 55, 37-48 CODEN: CBCOCH; ISSN: 1476-9271, 2015, XP029176088, DOI: 10.1016/J.COMPBIOLCHEM.2015.02.011 10.1016/J.COMPBIOLCHEM.2015.02.011
- ROBERT FRANKE ET AL: "Applied Hydroformylation", CHEMICAL REVIEWS, Bd. 112, Nr. 11, 14. November 2012 (2012-11-14), Seiten 5675-5732, XP055091930, ISSN: 0009-2665, DOI: 10.1021/cr3001803

## Beschreibung

Die Erfindung betrifft Phosphite mit einem Dihydroxyterphenyl mit einem Tetraphenyldioxaphospholan, sowie deren Verwendung als Liganden in der Hydroformylierung.

Phosphorhaltige Verbindungen spielen als Liganden in einer Vielzahl von Reaktionen eine entscheidende Rolle. Hierzu zählen Phosphitliganden, also Verbindungen, die P-O-Bindungen enthalten, die in der Hydrierung, Hydrocyanierung und vor allem in der Hydroformylierung Anwendung finden.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor PIII. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

A. Gangopadhyay offenbart in Computational Biology and Chemistry 2015, 55, 37-48 die Verbindung [2-[3-(2-phosphonooxyphenyl)phenyl]phenyl]phosphat als Cholera Toxin Inhibitor. EP3029056 offenbart Biphylen-verbrückte Bisphosphite und EP3029047 offenbart Terphenyl-2-oxy-Monophosphite. Die letzten beiden Dokumente offenbaren die Phosphit-Verbindungen als Liganden in Katalysatoren der Hydroformylierungsreaktion.

Der
Erfindung lag die Aufgabe zugrunde, neue Verbindungen bereitzustellen, welche in einem Verfahren zur Hydroformylierung von Olefinen eingesetzt werden können. Des Weiteren soll ein Verfahren zur Hydroformylierung von Olefinen bereitgestellt werden, welches zu einer guten Ausbeute an Aldehyd führt.

Gelöst wird die Aufgabe durch eine Verbindung gemäß Anspruch 1, beziehungsweise durch ein Verfahren gemäß Anspruch 4.

Verbindung gemäß der Formel (**2**):

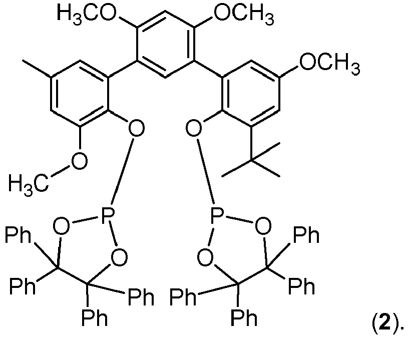

Neben der Verbindung wird auch ein Komplex beansprucht, welcher die Verbindung umfasst.

Komplex umfassend:
- die zuvor beschriebene Verbindung,
- ein Metallatom ausgewählt aus: Rh, Ru, Co, Ir.

Hierbei ist Rh bevorzugt.

Des Weiteren wird die Verwendung der zuvor beschriebenen Verbindung als Ligand in einem Ligand-Metall-Komplex zur Katalyse einer Hydroformylierungsreaktion beansprucht.

Verwendung der zuvor beschriebenen Verbindung als Ligand in einem Ligand-Metall-Komplex zur Katalyse einer Hydroformylierungsreaktion.

Neben der Verbindung an sich wird auch ein Verfahren zur Hydroformylierung von Olefinen beansprucht, in welchem diese Verbindung zum Einsatz kommen.

Verfahren umfassend die Verfahrensschritte:
a) Vorlegen eines Olefins,
b) Zugabe eines zuvor beschriebenen Komplexes,
   oder der zuvor beschriebenen Verbindung und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

In einer Variante des Verfahrens ist das Metallatom Rh.

In einer Variante des Verfahrens umfasst die Substanz (= Komplexvorstufe) Cyclooctadien.

In einer Variante des Verfahrens handelt es sich bei der Substanz (= Komplexvorstufe) um [(acac)Rh(COD)].

In einer Variante des Verfahrens wird das Reaktionsgemisch im Verfahrensschritt d) auf eine Temperatur im Bereich von 100 ° C bis 140 °C erwärmt.

Im Folgenden wird die Erfindung anhand von Ausführungsbeispielen näher erläutert.

### Experimentelles

Alle präparativen Arbeiten erfolgten unter Anwendung der Schlenk-Technik unter Argon als Schutzgas. Trockenes und luftfreiese Toluol und Tetrahydrofuran wurden mittels eines Pure Solv. MD-7 Systems erhalten und unter Argon aufbewahrt. Triethylamin wurde vor dem Einsatz unter Argon von Natriumketyl destilliert. Phosphortrichlorid (Aldrich) wurde vor dem Einsatz unter Argon destilliert. Alle präparativen Arbeiten erfolgten in ausgeheizten Gefäßen. Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Vorstufe:

Die Vorstufe wurde analog zu der folgenden Versuchsbeschreibungen A) synthetisiert.

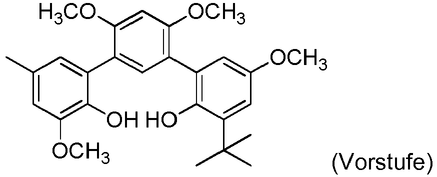

### Elektrochemische Kreuzkupplung

### Reaktionsschema

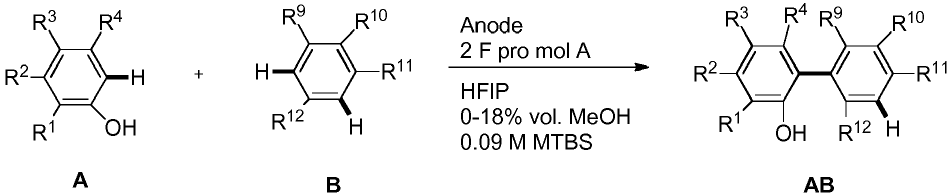

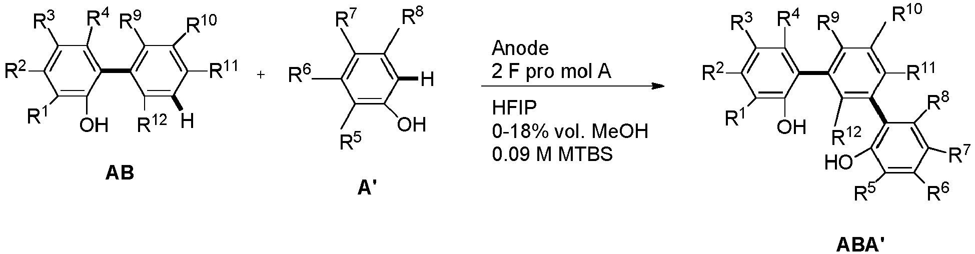

### Allgemeine Arbeitsvorschrift

5 mmol der zu oxidierenden Spezies A bzw. A' (Unterschusskomponente) wurden mit einem 2-3 fachen Überschuss (10-15 mmol) des Kupplungspartners B bzw. AB in 33 mL 1,1,1,3,3,3-Hexafluorisopropanol (HFIP) bzw. 33 mL (HFIP mit 18 Vol% Methanol (MeOH) bezogen auf die Summe aus HFIP und MeOH) in einer ungeteilten Flanschzelle mit BDD-Anode und Nickelnetzkathode umgesetzt. Als Leitsalz verwendete man BU₃NMe⁺MeOSO₃⁻ (MTBS) mit einer Konzentration von 0.09 M. Die Elektrolyse erfolgte galvanostatisch. Der Außenmantel der Elektrolysezelle temperierte man über einen Thermostaten auf etwa 10 °C, während die Reaktionsmischung gerührt und mit Hilfe eines Ölbades auf 50 °C erhitzt wurde. Nach Ende der Elektrolyse wurde der Zellinhalt in einen 50 mL Rundhalskolben überführt und das Lösungsmittel unter vermindertem Druck am Rotationsverdampfer bei 50 °C, 200 - 70 mbar entfernt. Mineralisationsprodukte sowie das enthaltene Leitsalz trennte man durch Elution mittels Essigsäureethylester (300 mL) über 50 g Kieselgel 60 ab. Nicht umgesetztes Edukt wurde mittels Kurzwegdestillation an der Kugelrohrdestille zurückerhalten (100 °C, 10⁻³ mbar). Die entstandenen Reaktionsprodukte wurden wie jeweils angegeben säulenchromatographisch getrennt.

### Elektrodenmaterial:

| | |
|---|---|
| Anode: | BDD (15 µm Diamantschicht) auf Siliciumträger |
| Kathode: | Nickelnetz |

### Elektrolysebedingungen:

| | |
|---|---|
| Temperatur: | 50 °C |
| Stromdichte: | 2,8 mA/cm² |
| Ladungsmenge: | 2 - 4 F bezogen auf die Unterschusskomponente |
| Klemmspannung: | 3 - 6V |

### A) 2,2"-Dihydroxy-4",5-dimethyl-2',3,4',5"-tetramethoxy[1,1';5',1"]terphenyl

Die Elektrolyse wurde gemäß der allgemeine Arbeitsvorschrift mit 250 mg (1.8 mmol, 1.0 Äquiv.) 4-Methoxy-3-methylphenol und 1.49 g (5.4 mmol, 3.0 Äquiv.) 2-Hydroxy-5-methyl-2',3,4'-trimethoxybiphenyl durchgeführt. Die Stromdichte betrug 2.8 mA/cm², die Ladungsmenge 2 F pro 4-Methoxy-3-methylphenol (Q = 350 C). Erste Aufreinigung erfolgte durch Abtrennung der Edukte an der Kugelrohrdestille (120 °C, 10⁻³ mbar). Das Produkt wurde säulenchromatographisch über Kieselgel 60 mit einem Gradienten 4:1, dann 7:3, dann 2:1 (CH:EE) aufgereinigt. Die Terphenylverbindung wurde als gelblicher Feststoff erhalten. 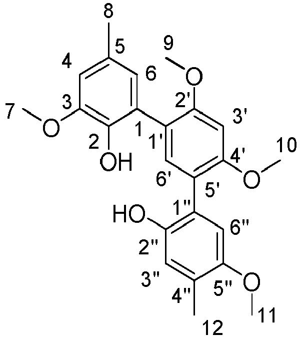
Ausbeute: 126 mg (0,3 mmol, 17%)
GC (Methode *hart,* HP-5): t_{R} = 23,54 min
R_{f}(Cy:EE = 2:1) = 0,14
¹H-NMR (400 MHz, CDCb) δ [ppm] = 2.25 (s, 3H), 2.34 (s, 3H), 3.79 (s, 3H), 3.93 (s, 3H), 3.93 (s, 3H), 3.97 (s, 3H), 5.57 (bs, 2H), 6.76 - 6.70 (m, 4H), 6.87 (s, 1H), 7.35 (s, 1H).
¹³C-NMR (101 MHz, CDCl₃) δ [ppm] = 16.06, 21.22, 56.06, 56.09, 56.37, 56.66, 77.36, 96.56, 111.26, 112.87, 119.82, 119.93, 120.96, 123.04, 123.75, 124.46, 127.65, 129.24, 135.01, 141.03, 147.08, 152.09, 156.00, 157.29.
MS (ESI, pos. mode): *m*/*z* für C₂₄H₂₆O₆ (M+Na⁺): berechnet: 433.16; gefunden: 433.16

### Liganden

### a) 6,6'-((3-(tert-Butyl)-3",4',5,6'-tetramethoxy-5"-methyl-[1,1':3',1"-terphenyl]-2,2" diyl)bis(oxy))didibenzo[d,f][1,3,2]dioxaphosphepin(Vergleichsligand 1)

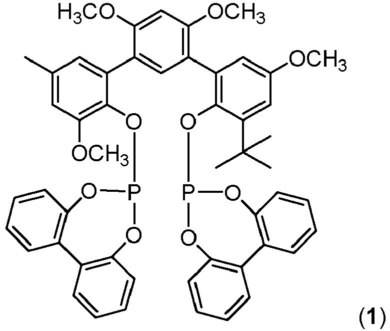

Eine gerührte Lösung von 6-Chlordibenzo[*d,f*][1,3,2]dioxaphosphepin (0,671 g; 2,677 mmol) in Toluol (4 ml) wird bei 0 °C tropfenweise mit einer Mischung aus dem Dihydroxyterphenyl (0,404 g; 0,892 mmol) und Triethylamin (0,39 ml) in Toluol (10 ml) versetzt. Man rührt über Nacht bei Raumtemperatur und anschließend 12 h bei 70 °C Badtemperatur. Es wird filtriert. Zum auf 0 °C gekühlten Filtrat gibt man tropfenweise eine Mischung aus 2,4-Di-*tert*.-butylphenol (0,186 g, 0,9 mmol) und Triethylamin (0,2 ml), man lässt auf Raumtemperatur kommen und rührt über Nacht. Die Reaktionslösung wird filtriert, das Filtrat wird im Vakuum zur Trockne eingeengt, der erhaltene Rückstand mit Heptan (15 ml) gewaschen und das so erhaltene Produkt aus Dichlormethan/Heptan bei -24 °C umkristallisiert. Ausbeute: 0,114 g (0,129 mmol, 15%).

Elementaranalyse (ber. für C₅₁H₄₆O₁₀P₂ = 880,87 g/Mol): C 69,30 (69,54); H 5,40 (5,26); P 7,03 (7,03)%.
³¹P-NMR (CD₂Cl₂): 146,9 (s); 150,1 (s) ppm.
¹H-NMR (CD₂Cl₂): 1,57 (s, 9 H); 2,34 (s, 3H); 3,69 (s, 3H); 3,89 (s, 3H); 3,94 (s, 3H); 4,04 (s, 3H); 6,72 (m, 2H); 6,79 (m, 1H); 6,92 (m, 1H); 6,96 (m, 2H); 7,03 (d, *J*_{HH} = 3,3 Hz; 1H); 7,11 (m, 2H); 7,27-7,41 (m, 9H); 7,51-7,55 (m, 4H) ppm.
ESI-TOF HRMS: *m*/*e* 902,24536 (M+Na)⁺.

### b) 2,2'-((3-(tert-Butyl)-3",4',5,6'-tetramethoxy-5"-methyl-[1,1':3',1"-terphenyl]-2,2"-diyl)bis(oxy)) bis (4,4,5,5-tetraphenyl-1,3,2-dioxaphospholan) (Ligand 2)

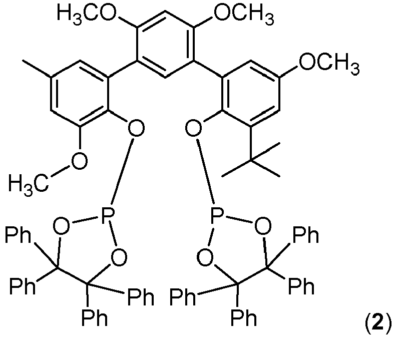

Zu einer gerührten Lösung des Dihydroxyterphenyls (0,266 g; 0,588 mmol) in THF (3 ml) wird bei -20 °C tropfenweise eine 0,32 M Lösung von n-Butyllithium in Hexan (3,68 ml; 1,176 mmol) gegeben. Man rührt 20 min bei dieser Temperatur nach, lässt auf Raumtemperatur kommen und addiert eine Lösung von 2-Chlor-4,4,5,5-tetraphenyl-1,3,2-dioxaphospholan (0,507 g; 1,176 mmol) in THF (3 ml). Die Reaktionslösung wir über Nacht gerührt, filtriert, und das Filtrat im Vakuum zur Trockne eingeengt. Das Rohprodukt wird zunächst säulenchromatografisch vorgereinigt (Hexan/Ethylazetat, 2:1, R*_{f}* = 0,5) und dann mit Heptan aus einer Lösung in THF gefällt. Ausbeute: 0,420 g (0,338 mmol; 57%).
Elementaranalyse (ber. für C₇₉H₇₀O₁₀P₂ = 1241,36 g/mol): C 75,68 (76,44); H 5,88 (5,68); P 4,23 (4,99)%.
³¹P-NMR (CD₂Cl₂): 144,6 (s); 146,6 (s) ppm.
ESI-TOF HRMS: *m*/*e* 1263,43281 (M+Na)⁺.

### Durchführung der Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl behandelt und unter Argon destilliert. Das als Substrat eingesetzte Olefin *cis*/*trans*-2-Penten (Aldrich) wurde mehrere Stunden über Natrium am Rückfluß erhitzt und unter Argon destilliert.
Für die Versuche wurden im Autoklaven unter Argonatmosphäre Lösungen der Katalysatorvorstufe und des Liganden gemischt. Als Komplexvorstufe (= Katalysatorvorstufe) kam [(acac)Rh(COD)] (Umicore, acac=Acetylacetonat-Anion; COD=1,5-Cyclooctadien) zum Einsatz. Für Versuche mit einer Konzentration von 100 ppm-m Rhodium wurden 10 ml einer 4,31 millimolaren Lösung in den Autoklaven gegeben. Anschließend wurde die entsprechende Masse des Liganden in 10 ml Toluol gelöst und zugemischt. Durch Zugabe von weiterem Toluol wurde das Anfangsvolumen der Katalysatorlösung auf 41,0 ml eingestellt. In eine druckfeste Pipette wurde als Olefin eingefüllt: 2-Penten 9,75 g; 139,00 mmol. Der Autoklav wurde bei einem Gesamtgasdruck (Synthesegas: Linde; H₂ (99,999%) : CO (99,997%) = 1:1) von 12 bar für den Enddruck von 20 bar unter Rühren (1500 U/min) aufgeheizt. Nach Erreichen der Reaktionstemperatur von 120 °C wurde der Synthesegasdruck auf 19,5 bar für einen Enddruck von 20 bar erhöht und das Olefin(-gemisch) mit einem in der Druckpipette eingestellten Überdruck von ca. 3 bar in den Autoklaven gepresst. Die Reaktion wurde bei konstantem Druck von 20 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm.

Die Ergebnisse der Hydroformylierungsversuche sind in der nachfolgenden Tabelle zusammengestellt:

**Tabelle 1:**

| **Substrat: 2-Penten, 20 bar CO/H₂** | | | | |
|---|---|---|---|---|
| **Ligand** | **p [bar]** | **T [°C]** | **L/Rh** | **Ausbeute Aldehyd (%)** |
| **1** | 20 | 120 | 2 | 56 |
| **2*** | 20 | 120 | 2 | 98 |

| | | | | |
|---|---|---|---|---|
| * erfindungsgemäße Verbindung bzw. erfindungsgemäßes Verfahren Reaktionsbedingungen: *t* = 4h, [Rh] = 100 ppm-m, Lösungsmittel: Toluol. | | | | |

Wie die Versuchsergebnisse zeigen, wird die Aufgabe durch das erfindungsgemäße Verfahren gelöst.

## Patentansprüche

1. Verbindung gemäß der Formel (**2**):

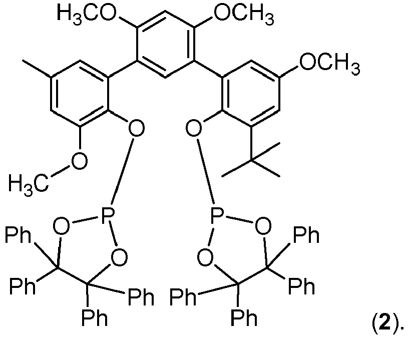

2. Komplex umfassend:
- eine Verbindung nach Anspruch 1;
- ein Metallatom ausgewählt aus: Rh, Ru, Co, Ir.

3. Verwendung einer Verbindung nach Anspruch 1,
als Ligand in einem Ligand-Metall-Komplex zur Katalyse einer Hydroformylierungsreaktion.

4. Verfahren umfassend die Verfahrensschritte:
a) Vorlegen eines Olefins,
b) Zugabe eines Komplexes nach Anspruch 2,
oder einer Verbindung nach Anspruch 1 und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

5. Verfahren nach Anspruch 4,
wobei das Metallatom Rh ist.

## Claims

1. Compound according to the formula (**2**):

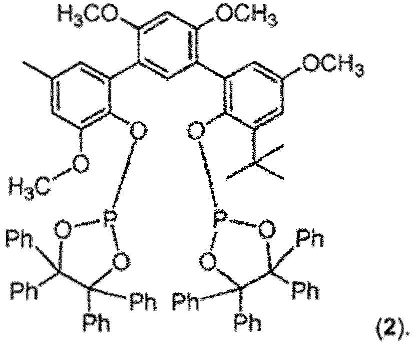

2. Complex comprising:
- a compound according to Claim 1
- a metal atom selected from: Rh, Ru, Co, Ir.

3. Use of a compound according to Claim 1 as ligand in a ligand-metal complex for catalysing a hydroformylation reaction.

4. Process comprising the process steps of:
a) providing an olefin,
b) adding a complex according to Claim 2,
or a compound according to Claim 1 and a substance comprising a metal atom selected from: Rh, Ru, Co, Ir,
c) feeding H₂ and CO,
d) heating the reaction mixture, wherein the olefin is converted to an aldehyde.

5. Process according to Claim 4,
wherein the metal atom is Rh.

## Revendications

1. Composé selon la formule (2) :

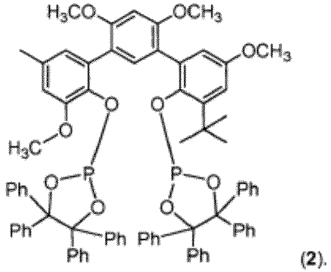

2. Complexe comprenant :
- un composé selon la revendication 1 ;
- un atome métallique choisi parmi : Rh, Ru, Co, Ir.

3. Utilisation d'un composé selon la revendication 1 comme ligand dans un complexe ligand-métal pour la catalyse d'une réaction d'hydroformylation.

4. Procédé comprenant les étapes de procédé :
a) disposition préalable d'une oléfine,
b) addition d'un complexe selon la revendication 2,
ou d'un composé selon la revendication 1 et d'une substance qui présente un atome métallique choisi parmi : Rh, Ru, Co, Ir,
c) introduction de H₂ et de CO,
d) chauffage du milieu réactionnel, l'oléfine étant transformée en aldéhyde.

5. Procédé selon la revendication 4, l'atome métallique étant Rh.
